Europäisches Patentamt

European Patent Office

Office européen des brevets

(19)

(11) **EP 0 600 333 B1**

(12) **EUROPÄISCHE PATENTSCHRIFT**

(45) Veröffentlichungstag und Bekanntmachung des Hinweises auf die Patenterteilung:
**12.03.1997 Patentblatt 1997/11**

(51) Int Cl.⁶: **C07C 57/04**, C07C 51/215, C07C 47/22, C07C 45/33

(21) Anmeldenummer: **93118726.4**

(22) Anmeldetag: **22.11.1993**

(54) **Katalytische Oxidation von Isobutan zu Methacrylsäure und Methacrolein**

Catalytic oxidation of isobutane to methacrylic acic and methacrolein

Oxydation catalytique d'isobutane en acide méthacrylique et en méthacroleine

(84) Benannte Vertragsstaaten:
**AT BE CH DE DK ES FR GB IT LI NL SE**

(30) Priorität: **28.11.1992 DE 4240085**

(43) Veröffentlichungstag der Anmeldung:
**08.06.1994 Patentblatt 1994/23**

(73) Patentinhaber: **Röhm GmbH**
**64293 Darmstadt (DE)**

(72) Erfinder:
• **Bielmeier, Ernst, Dr.**
**D-64347 Griesheim (DE)**
• **Haeberle, Thomas, Dr.**
**D-64683 Einhausen (DE)**
• **Siegert, Hermann-Josef, Dr.**
**D-64342 Seeheim-Jugenheim (DE)**
• **Gruber, Wilhelm, Dr.**
**D-64287 Darmstadt (DE)**

(56) Entgegenhaltungen:
EP-A- 0 005 769          EP-A- 0 194 541
EP-A- 0 284 947          EP-A- 0 510 566
US-A- 5 191 116

**Beschreibung**

Die Erfindung betrifft die Herstellung von Methacrylsäure und Methacrolein durch Gasphasenoxidation von Isobutan an Molybdän-Heteropolysäure-Katalysatoren.

Stand der Technik

Methacrylsäure läßt sich durch Gasphasenoxidation verschiedener Ausgangsstoffe wie Isobutylen, Methacrolein, Isobuttersäure und Isobutan herstellen. Die Oxidationsreaktionen mit molekularem Sauerstoff werden an Festkörperkatalysatoren unter Beibehaltung der molekularen Kohlenstoffanordnung selektiv gelenkt. Fast ausnahmslos alle diese Feststoffkatalysatoren enthalten als wesentlichen Bestandteil Molybdän in oxidischer Bindung neben weiteren, Aktivität und Selektivität der Katalysatoren beeinflussenden Elementen. Besondere Bedeutung kommt Katalysatoren zu, die auf Basis von Heteropolysäuren des Molybdäns mit Phosphor als Zentralatom hergestellt bzw. aufgebaut sind.

So können nach der DE-OS 27 22 375 z.B. $H_5PMo_{10}V_2O_{40}$-Heteropolysäure-haltige Katalysatoren, u.a. auch Kupferhaltig, sowohl für die Oxidation von Methacrolein zu Methacrylsäure als auch für die Oxidehydrierung von Isobuttersäure oder deren Ester zu Methacrylsäure oder deren Ester eingesetzt werden. Nach dem EP-B 0 005 769 werden Katalysatoren der Formel $Mo_aV_bP_cX_dO_e$, die Heteropolysäurestruktur haben, zur Oxidation von Isobutylen und/oder tert.-Butanol zu Methacrolein und Methacrylsäure verwendet. Ein Verfahren zur Oxidation von Methacrolein zu Methacrylsäure an Katalysatoren der gleichen Art ist in der DE-OS 30 10 434 beschrieben.

Die Heteropolysäure der Formel $H_8PMo_{10}VO_{39}$ und deren Anhydrid $PMo_{10}VO_{35}$ sind nach EP-B 0 194 541 als temperaturstabilere Heteropolysäure-Katalysatoren in Oxidationsreaktionen, wie Oxidationen der Olefine Propylen und Isobutylen zu den entsprechenden ungesättigten Aldehyden Acrolein und Methacrolein und auch zu deren Weiteroxidation zu Acrylsäure und Methacrylsäure, und in Oxidehydrierungsreaktionen von Isobuttersäure oder ihrer Ester zu Methacrylsäure oder ihrer Ester geeignet. Nach der EP-B 0 113 084 sind Kupferderivate dieses Heteropolysäureanhydrids, z.B. $Cu_{0,2}PMo_{10}VO_{35,2}$ und auch Kupferderivate von anderen Heteropolysäuren, wie der $H_5PMo_{10}V_2O_{40}$-Heteropolysäure, sehr selektive Katalysatoren bei der Oxidehydrierung von Isobuttersäure zu Methacrylsäure.

In der EP-A 284 947 ist die Herstellung von Molybdän-Heteropolysäure-Oxidationskatalysatoren beschriebener Art angegeben, wobei wasserlösliche, praktisch nichtflüchtige organische Verbindungen, insbesondere Polymere, mitverwendet werden, die dann bei 150 bis 400 Grad C in Gegenwart von Sauerstoff calciniert werden. So hergestellte Katalysatoren zeichnen sich bei der Oxidehydrierung von Isobuttersäure zu Methacrylsäure durch ein verbessertes Langzeitverhalten aus.

Weiter wird in der EP-A 0 376 117 ein Verfahren zu Herstellung von Methacrylsäure durch Oxidation von Methacrolein an oxidischen Katalysatoren der Formel $Mo_{12}P_aV_bCs_cAs_dCu_eX_fY_gO_x$ vorgeschlagen, denen bei ihrer Formgebung neben bekannten Kohlenstoff-haltigen Verbindungen als Gleitmittel noch Formhilfsmittel und Verstärkungsmittel wie Microfasern aus anorganischen Materialien wie beispielsweise Glas oder Asbest zugesetzt werden können, und die bei Temperaturen von 180 bis 480 Grad C, gegebenenfalls in Luftatmosphäre, calciniert sind.

Katalysatoren für selektive Oxidationen haben im allgemeinen eine kleine innere Oberfläche, d.h. das Katalysatorkorn ist mit relativ wenigen Poren versehen, oder aber sie werden mit porösen Trägern mit weiträumigen Poren im Inneren des Trägermaterials hergestellt, in die das katalytische Material dann eingebettet ist. Solche Träger sind für einen Diffusionsstrom zum Materie- und Energietransport an das katalytisch aktive Material gut durchlässig. Kombinationen von Molybdän-, Vanadium-, Phosphor- und Sauerstoff-haltigen Stoffen und Trägern mit einer Porosität von 10 bis 80 % und einer inneren Oberfläche unter 1 $m^2$/g sind als Katalysatoren für die Oxidehydrierung von Isobuttersäure zu Methacrylsäure in der DE-OS 31 45 091 beschrieben.

Schließlich sind in der DE-OS 41 13 423 Oxidationskatalysatoren mit Molybdän, Phosphor und Vanadium als wesentlichen Elementen in oxidischer Form und Heteropolysäure-Basis beschrieben, die Kanäle enthalten, welche durch Herausbrennen von organischen Fasern gebildet sind. Sie werden verwendet zur Oxidation von Propylen oder Isobutylen zu Acrolein bzw. Methacrolein, zu deren Weiteroxidation zu den entsprechenden ungesättigten Säuren - (Meth)acrylsäure - und zur Oxidehydrierung von Isobuttersäure oder ihrer niederen Ester zu Methacrylsäure oder deren Ester.

Die Oxidation von Isobutan zu Methacrylsäure und Methacrolein an oxidischen Feststoffkatalysatoren, die Molybdän, Antimon und Phosphor enthalten, ist aus EP-A 0 010 902 bekannt, wonach bei ca. 10 %igem Isobutanumsatz Methacrylsäure in ca. 50 %iger und Methacrolein in ca. 20 %iger Selektivität enthalten wird. Nach der JP 0 320 237 wird an einem Oxidkontakt mit Phosphor, Vanadium, Molybdän, Antimon und Kupfer Isobutan bei 9,5 %igem Umsatz in 51,4 %-iger Selektivität zu Methacrolein umgesetzt.

Auch Feststoffkatalysatoren, die aus Heteropolysäuren des Molybdäns und ihrer Salzderivate hergestellt sind, eignen sich zur selektiven Isobutanoxidation. Nach der EP-A 0 418 657 sind dies Phosphormolybdän-Heteropolysäuresalze, die zwingend mindestens ein Element von Rubidium, Caesium und Thallium, wahlweise Vanadium oder Arsen und gegebenenfalls z.B. Kupfer oder ein anderes Metall enthalten. In der EP-A 0 425 666 wird ein Verfahren zur Herstellung von Methacrylsäure und Methacrolein durch Oxidation von Isobutan beschrieben, das an Katalysatoren hergestellt aus Molybdänheteropolysäuren mit Phosphor und/oder Arsen als Zentralatom, die zwingend mindestens einen Bestandteil aus der Gruppe eines Alkalimetalls, eines Erdalkalimetalls und Thallium, weiter Vanadium und/oder Kupfer und wahlweise weitere metallische Elemente enthalten, durchgeführt wird.

Katalysatoren für die Isobutanoxidation, die aus Heteropolysäuren des Molybdäns mit Phosphor und/oder Arsen als Zentralatom und ohne Vanadium als Molybdän-ersetzendes koordinierendes Peripherieatom hergestellt sind, werden in den japanischen Patentanmeldungen JP 02 042 033, nach der weiter Kupfer obligatorisch enthalten sind, JP 02 042 034 und in JP 62 132 832 sowie JP 63 145 249, wobei die beiden zuletzt genannten Anmeldungen Verfahrensvarianten behandeln, beschrieben.

Aufgabe und Lösung

Obwohl die erzielbaren Ausbeuten an Endprodukt, der Umsatz des eingesetzten Ausgangsprodukts oder die erreichbare Raum-Zeit-Ausbeute wichtige Qualitätsmerkmale eines Katalysators bei einer bestimmten Umsetzung sind, ist die Selektivität, d.h. der Quotient aus Ausbeute und Umsatz, das wichtigste Qualitätsmerkmal eines Katalysators, denn sie gibt an, welcher Teil der umgesetzten Ausgangsstoffe tatsächlich in das gesuchte Endprodukt übergeführt werden.

Es bestand daher die Aufgabe, die Selektivität bei der Oxidation von Isobutan zu Methacrylsäure und Methacrolein durch Verwendung besserer Katalysatoren zu erhöhen.

Dieses Ziel wird durch Verwendung von Katalysatoren auf Basis von Heteropolysäuren des Molybdäns und Vanadiums mit Phosphor als Zentralatom erfindungsgemäß dadurch erreicht, daß diese ohne Anwesenheit weiterer kationischer Verbindungen, insbesondere ohne Anwesenheit von Alkalimetall- und Erdalkalimetallverbindungen, noch Kupfer enthalten oder diese die Kupfer-freie $H_8PMo_{10}VO_{39}$-Heteropolysäure oder deren Anhydrid $PMo_{10}VO_{35}$ sind.

Die Erfindung betrifft danach ein Verfahren zur Herstellung von Methacrylsäure und Methacrolein durch Oxidation von Isobutan mit molekularem Sauerstoff in der Gasphase bei Temperaturen von 250 bis 450 Grad C an einem Feststoffkatalysator, hergestellt aus Heteropolysäuren, die Phosphor als Zentralelement und Molybdän als ein koordinierendes Element enthalten,

dadurch gekennzeichnet,

daß die Katalysatoren aus Heteropolysäuren bzw. deren dehydratisierten Derivaten der Formel

$$Cu_aH_bP_cMo_dV_eO_f \tag{I}$$

mit

a = 0,1 bis 1, b = 0 bis 7,8, c = 0,8 bis 1,2, vor allem 1, d = 9 bis 12, vor allem 10 oder 11, e = 0,5 bis 3, vor allem 1 oder 2, f = abhängig von den Molzahlen a bis e,

oder/und der Formel

$$H_8PMo_{10}VO_{39} \tag{II}$$

bzw. deren Anhydrid

$$PMO_{10}VO_{35} \tag{III}$$

gebildet sind.

Mit den aus den Verbindungen I oder/und II bzw. III gebildeten Feststoffkatalysatoren lassen sich für die Summe Methacrylsäure und Methacrolein Selektivitäten von deutlich über 80 % und Spitzenwerte bis um 90 % erreichen, im Vergleich zu einem Maximalwert von 75 % wie in der EP-A 0 425 666 (Beispiel 2) angegeben.

Insbesondere läßt sich mit Katalysatoren, die aus den Verbindungen II bzw. III hergestellt sind, erfindungsgemäß die Oxidation von Isobutan bei Temperaturen über 350 Grad C durchführen, da diese Katalysatoren im Gegensatz zu anderen bekannten Heteropolysäurekatalysatoren, so auch zu den in EP-A 0 425 666 und in EP-A 0 418 657 verwendeten Katalysatoren thermisch stabiler sind.

Die Selektivitäten der Wertstoffe Methacrylsäure und Methacrolein lassen sich bei dem Verfahren weiter erhöhend beeinflussen, wenn die Katalysatoren mit den Heteropolysäurekomponenten I oder/und II bzw. III in Gegenwart verbrennbarer, porenbildender, faseriger Zusatzstoffe konfektioniert sind.

Der Umsatz von Isobutan ist unter anderem vom Molverhältnis Isobutan : Sauerstoff abhängig. Dieser und damit auch die Raum-Zeit-Ausbeuten von Methacrylsäure und Methacrolein lassen sich steigern durch Erhöhung der Isobutan-Eingangskonzentration oder des Sauerstoffanteils im umzusetzenden Gasgemisch, vorteilhaft durch Verwendung Sauerstoff-angereicherter Luft.

Durchführung der Erfindung

Aktive Komponente des Katalysators.
Die erfindungsgemäß verwendeten Katalysatoren enthalten als aktives Material Stoffe, die Molybdän, Phosphor, Vanadium und teilweise Kupfer als einen oxidischen Verband enthalten, die aus den Verbindungen (I) der chemischen Zusammensetzung $Cu_aH_bPMo_dV_eO_f$ mit den oben angegebenen Bedeutungen für a, b, c, d, e und f oder aus der $H_8PMo_{10}VO_{39}$ (II) oder aus $PMo_{10}VO_{35}$ (III) gebildet sind. Diese Katalysatorstoffe sind Vanadiumderivate der Phosphor-Molybdän-Heteropolysäuren, die sich durch die chemische Formel $H_{3+x}PMo_{12-x}V_xO40$ mit x = 1, 2, 3 beschreiben lassen und zwingend Kupfer enthalten oder sind die $H_8PMo_{10}VO_{39}$-Heteropolysäure oder deren Anhydrid $PMo_{10}VO_{35}$, sowie Kupferderivate dieser Heteropolysäure.

Zur Herstellung der im erfindungsgemäßen Verfahren verwendeten Katalysatoren können die Heteropolysäureverbindungen aus Oxiden der einzelnen Komponenten, wie z.B. $MoO_3$, $V_2O_5$, $H_3PO_4$, $CuO$, oder aus bei höheren Temperaturen zersetzlichen Salzen, wie $(NH_4)_2MoO_4$, $NH_4VO_3$, $Cu(NO_3)_2$, nach bekannten Verfahren erhalten werden. Solche Herstellungsweisen sind beispielsweise in der DE-OS 27 22 375 und in der EP-B 0 046 840 beschrieben. Das Ziel, selektivere Katalysatoren bei der Oxidation von Isobutan zu verwenden, wird vor allem dadurch erreicht, wenn die erfindungsgemäß zu verwendenden Heteropolysäuren I durch hydrothermische Umsetzung von oxidischen Verbindungen des Molybdäns, Vanadiums, Phosphors und Kupfers, wie insbesondere in der EP-B 0 113 084 beschrieben, und die Heteropolysäure II bzw. deren Anhydrid III hydrothermisch nach EP-B 0 194 541 hergestellt sind.

Der Begriff "hydrothermische Umsetzung" ist in der DE-OS 27 22 375 für die Bildung einer Mo-V-Phosphat-Heteropolysäure aus den Oxiden von Mo, V und P in wäßriger Phase bei 60 Grad C oder mehr während einer meist mehrstündigen Reaktionszeit verwendet worden. Anstelle der Oxide können auch Ammoniummolybdat oder Ammoniumvanadat zur Bildung von Heteropolysäuren verwendet werden. Allerdings haben sich Ammoniumverbindungen für die Katalysatoreigenschaften als ähnlich nachteilig wie Alkaliverbindungen erwiesen, so daß sie vorzugsweise nicht verwendet werden. Die Entstehung der Heteropolysäure verläuft besonders günstig in der Siedehitze unter Normaldruck und ist an einer allmählich tiefen Rotfärbung der wäßrigen Lösung zu erkennen.

Der Begriff "Heteropolysäure" soll hier nicht auf ganzzahlige stöchiometrische Verhältnisse beschränkt sein. In den reinen Heteropolysäuren liegt vor allem ein Mo : P-Verhältnis von 11 : 1 oder 10 : 1 vor. Es wurde jedoch festgestellt, daß auch etwas größere oder kleinere Mo : P-Verhältnisse zu guten Katalysatoren führen. Vermutlich liegen bei P-Unterschuß in der wäßrigen Phase neben den eigentlichen Heteropolysäuren auch Mo-Isopolysäuren vor. Das gesamte in Gegenwart von Cu hergestellte Säurengemisch wird hier als Heteropolysäure bezeichnet.

Beispiele für Kupfer-haltige Zusammensetzungen der Formel I sind $Cu_{0,2}H_{3,6}PMo_{11}VO_{40}$, $Cu_{0,4}H_{4,2}PMo_{10}V_2O_{40}$, $Cu_{0,2}H_{7,6}PMo_{10}VO_{39}$, $CuH_6PMo_{10}VO_{39}$.

Konfektionierung des Katalysators

Die Cu-haltige Mo-V-P-Heteropolysäurelösung oder/und die $H_8PMo_{10}VO_{39}$-Lösung kann als solche in Abwesenheit von Feststoffen eingetrocknet und der Rückstand kalziniert und und als Katalysator eingesetzt werden. Häufig wird es vorgezogen, den so erhaltenen Katalysator in einem Gewichtsverhältnis von z.B. 1 zu 0,1 bis 10 mit einem inerten Material, wie feinteiligem $SiO_2$, $Al_2O_3$, $ZrO_2$, Siliciumcarbid und dergl., zu verschneiden und zu einem körnigen Kontaktmaterial zu verarbeiten, was durch Extrusion, Pressen, Pelletieren oder Granulieren geschehen kann. Das Gemisch kann zu diesem Zweck mit Wasser oder wäßrigen Lösungen angeteigt werden. Auch kann man beim Pelletieren von harten, mehr oder weniger unporösen inerten Trägerkörpern, vorzugsweise mit rauher Oberfläche ausgehen und den Katalysator als dünne Schale nach der Dragiertechnik aufbringen. Dazu werden auf die Trägerkörper, z .B. keramische Kügelchen, in einer in Schräglage rotierenden Trommel der pulverförmige Katalysator und eine gegebenenfalls bindemittelhaltige Flüssigkeit aufgetragen und die entstehenden Pellets anschließend getrocknet. Erforderlichenfalls wird der ganze Arbeitsgang mehrfach wiederholt.

Die wäßrige Lösung der Heteropolysäure kann auch in Gegenwart eines festen Katalysatorträgermaterials eingetrocknet werden. Dazu eignen sich die oben schon genannten feinteiligen inerten Materialien. Die Trägerstoffe können eine Porosität unter 10 Vol.-% oder/und eine innere Oberfläche von vorteilhaft 0,1 bis 10 qm/g haben. Der Anteil dieser Träger kann, bezogen auf das Gesamtgewicht des Katalysators, 5 bis 95 Gew.-% ausmachen. Das Trägermaterial wird beispielsweise mit der Heteropolysäurelösung zu einem Brei vermischt und durch Erhitzen auf den Siedepunkt, gegebenenfalls bei Unterdruck bei Temperaturen von z.B. 50 bis 110 Grad C zur Trockne eingedampft oder poröse große Trägerpartikel, mit beispielsweise 2 bis 10 mm Kantenlänge oder Durchmesser, werden mit der Lösung der Heteropolysäure getränkt, wodurch Katalysatoren mit bis etwa 50 Gew.-% katalytisch aktivem Material in den Partikeln erhalten werden.

Für die Erzeugung der Kanäle in den erfindungsgemäß zu verwendenden Katalysatoren sind, wie in EP-A 0 510 566 beschrieben, sowohl natürliche als auch synthetische organische Fasern (Textilfasern) und Kohlenstoff-Fasern, die ihrerseits durch Verkohlung und Graphitisierung von natürlichen und synthetischen organischen, d.h. aus Kohlenstoffverbindungen bestehenden Fasern, entstehen, brauchbar. Diese werden, bei der Calcinierung im Temperaturbereich von 100 bis 380 Grad C, insbesondere in Gegenwart von Sauerstoff zerstört, und letztlich zu gasförmigen Produkten unter Hinterlassung von Hohlräumen, die praktisch den Dimensionen der vorher vorhandenen Fasern entsprechen, abgebaut. Als Naturfasern können beispielsweise Baumwolle, Wolle und Cellulosederivate und als organische Fasern solche aus Polyamiden (Nylon), Polyester, Polyolefinen oder Acrylnitril verwendet werden.

Die zu verwendenden Fasern haben Durchmesser von 1 bis 100 µm, insbesondere solche von 5 bis 70 µm, vor allem solche von 15 bis 50 µm und Längen, die auch das Mehrfache des Durchmessers des endgültigen Katalysatorteilchens sein können, wie beispielsweise 1 bis 30 mm, vorzugsweise 1 bis 10 mm, insbesondere von 2 bis 8 mm, vor allem von 3 bis 6 mm, und werden in Mengen von 0,5 bis 5 Gew.-%, insbesondere von 1 bis 4 Gew.-% und vor allem von 1,5 bis 3,5 Gew.-% bezogen auf das im Katalysator vorhandene, katalytisch aktive Material, zugesetzt.

Die Oxidation von Isobutan

wird an den vorgehend beschriebenen Katalysatoren mit molekularem Sauerstoff in einem Temperaturbereich von 250 bis 450 Grad C, insbesondere von 300 bis 450 Grad C, vorteilhaft bei 320 bis 400 Grad C, durchgeführt. Das Eingangsgas enthält pro Mol Isobutan 0,2 bis 10 Mol, insbesondere 0,5 bis 5 Mol Sauerstoff, beispielsweise in Form von Luft oder in Form von Sauerstoff-angereicherter Luft, außer Stickstoff gegebenenfalls weitere Inertgase, wie insbesondere CO und $CO_2$, vor allem durch im Kreislauf geführte Reaktionsgase, und weiter noch Wasserdampf, der in Mengen von 0 bis 10 Mol Pro Mol Isobutan vorhanden sein kann.

Die Reaktion kann in einem weiten Druckbereich, von Unterdruck bis Überdruck, d.h. von etwa 0,1 bis 10 atm, vor allem von 0,5 bis 5 atm, insbesondere von 0,8 bis 3 atm durchgeführt werden.

Als Oxidationsreaktoren werden insbesondere Festbettreaktoren, in Form von Rohrreaktoren verwendet. Die Oxidation kann aber auch in Reaktoren mit bewegtem Katalysatorbett und in Reaktoren mit Wirbelschichtkatalysatorführung durchgeführt werden.

Methacrylsäure und Methacrolein werden aus dem Reaktionsgas nach bekannten Methoden, wie der Kondensation, der Extraktion und der Destillation gewonnen und gereinigt. Nicht umgesetztes Isobutan wird wieder in den Oxidationsprozeß zurückgeführt, vorteilhafterweise mit anderen gasförmigen, vor allem inerten Verbindungen wie CO, $CO_2$, Stickstoff und Wasserdampf (Kreisgasführung).

Für die Herstellung von Methacrylsäure als wesentlichem Wertprodukt, ist es dann weiter vorteilhaft, gebildetes Methacrolein wieder zusammen mit Isobutan über den Katalysator zu schicken, da die erfindungsgemäß verwendeten Heteropolysäurekatalysatoren, wie im Stand der Technik beschrieben, bekannte und effektive Katalysatoren für die Oxidation von Methacrolein zu Methacrylsäure sind. Die Herstellung von Methacrylsäure aus Isobutan läßt sich danach in einem Reaktorsystem technisch durchführen.

Die Verweilzeit und damit der Kehrwert der Katalysatorbelastung wird durch den Quotienten $\frac{W}{F}$ mit der Dimension Stunde (h) quantifiziert, wobei W (Weight) das Gewicht der katalytisch aktiven Masse und F (Feed) das Gewicht der pro Stunde umzusetzenden Wertsubstanz, wie des Isobutans, gegebenenfalls mit Methacrolein in gleichen Gewichtseinheiten bedeutet.

In den folgenden Beispielen wird das erfindungsgemäße Verfahren mit verbesserten, vor allem selektiveren P-Mo-V-haltigen Heteropolysäure-Katalysatoren beschrieben.

Als Ergebnisse sind die ermittelten Selektivitäten (Sel.) für Methacrylsäure (MAA) und Methacrolein und die Raum-Zeit-Ausbeuten dieser Oxidationsprodukte angegeben.

BEISPIELE

A. Ein $SiO_2$-Träger wurde mit 43 % $Cu_{0,2}H_{3,6}PMo_{11}VO_{40}$ als katalytisch wirksamen Material belegt und bei 300 Grad C 4 Stunden lang in Luft calciniert. Anschließend wurde der Katalysator bei der Oxidation von Isobutan in der Gasphase eingesetzt. Die Komponenten des über diesen Katalysator geleiteten gasförmigen Gemisches aus Isobutan, Sauerstoff, Stickstoff und Wasserdampf wurden in bestimmten molaren Verhältnissen eingesetzt. Diese Verhältnisse sind in Tabelle 1 ebenso zusammengestellt wie die eingestellten Verweilzeiten (Weight/Feed = W/F (h)).

TABELLE 1

| | W/F (h) | Molverhältnis | | | |
|---|---|---|---|---|---|
| | | Isobutan: | $O_2$: | $N_2$: | $H_2O$ |
| Bedingung 1 | 4,1 | 1,15 | 4,5 | 6,55 | 3 |
| Bedingung 2 | 2,1 | 2,3 | 4,5 | 5,4 | 3 |
| Bedingung 3 | 1,0 | 4,6 | 4,5 | 3,1 | 3 |

TABELLE 2

Ergebnisse bei der Oxidation von Isobutan an $Cu_{2,0}H_{3,6}PMo_{11}VO_{40}$ (Bedingungen lt. TABELLE 1)

| Beisp. Nr. | Bedingung | Temperatur (Grad C) | Umsatz Isobutan (%) | MAA Sel. (%) | MAA RZA (g/L·h) | Methacrolein Sel. (%) | Methacrolein RZA (g/L·h) | Gesamt-selektivität (%) |
|---|---|---|---|---|---|---|---|---|
| 1 | 1 | 327,5 | 5,3 | 56,6 | 4,3 | 23,6 | 1,43 | 80,2 |
| 2 | 2 | 331,2 | 9,5 | 59,3 | 18,8 | 17,0 | 3,7 | 86,3 |
| 3 | 2 | 347,9 | 13,0 | 55,6 | 20,65 | 11,5 | 3,42 | 67,1 |
| 4 | 3 | 328,0 | 4,4 | 50,5 | 12,7 | 31,0 | 6,23 | 81,5 |

B. Als weiterer Katalysator wurde ein Kontakt, bestehend aus $SiO_2$-Träger und 45 % $H_8PMo_{10}VO_{39}$ als katalytisch

aktiver Komponente, bei der Oxidation von Isobutan eingesetzt. Bei den in Tabelle 3 angegebenen molaren Verhältnissen der gasförmigen Reaktanden wurden die in Tabelle 4 dargestellten Ergebnisse erzielt.

TABELLE 3

|  | W/F (h) | Molverhältnis | | | |
|---|---|---|---|---|---|
|  |  | Isobutan: | $O_2$: | $N_2$: | $H_2O$ |
| Bedingung 4 | 3 | 3 | 3 | 2 | 2 |
| Bedingung 5 | 3 | 3 | 3 | 0 | 2 |

TABELLE 4

Ergebnisse bei der Oxidation von Isobutan an $H_8PMo_{10}VO_{39}$ (Bedingung lt. Tabelle 3)

| Beisp. Nr. | Bedingung | Temperatur Grad C | Umsatz Isobutan (%) | MAA Sel. (%) | MAA RZA (g/L·h) | Methacrolein Sel. (%) | Methacrolein RZA (g/L·h) | Gesamt-selektivität (%) |
|---|---|---|---|---|---|---|---|---|
| 5 | 4 | 330,7 | 2,3 | 13,3 | 0,9 | 77,2 | 3,9 | 90,5 |
| 6 | 4 | 351,2 | 3,5 | 18,4 | 1,8 | 67,4 | 5,2 | 85,8 |
| 7 | 4 | 361,4 | 4,0 | 24,4 | 2,7 | 58,0 | 5,1 | 82,4 |
| 8 | 4 | 370,1 | 5,1 | 30,1 | 4,2 | 50,2 | 5,1 | 80,3 |
| 9 | 5 | 341,1 | 4,1 | 19,3 | 2,4 | 63,2 | 6,1 | 82,5 |
| 10 | 5 | 350,8 | 4,6 | 22,1 | 2,8 | 59,3 | 6,0 | 81,4 |
| 11 | 5 | 370,6 | 7,6 | 29,6 | 6,2 | 41,4 | 6,9 | 71,0 |

**Patentansprüche**

1. Verfahren zur Herstellung von Methacrylsäure und Methacrolein durch Oxidation von Isobutan mit molekularem Sauerstoff in der Gasphase bei Temperaturen von 250 bis 450 Grad C an einem Feststoffkatalysator, hergestellt aus Heteropolysäuren, die Phosphor als Zentralelement und Molybdän als ein koordinierendes Element enthalten, dadurch gekennzeichnet, daß die Katalysatoren aus Heteropolysäuren bzw. deren dehydratisierten Derivaten der Formel

$$Cu_aH_bP_cMo_dV_eO_f \qquad\qquad (I)$$

mit

a = 0,1 bis 1, b = 0 bis 7,8, c = 0,8 bis 1,2, d = 9 bis 12, e = 0,5 bis 3, f = abhängig von dem Molzahlen a bis e, oder/und der Formel

$$H_8PMo_{10}VO_{39} \qquad\qquad (II)$$

bzw. deren Anhydrid

$$PMo_{10}VO_{35} \qquad\qquad (III)$$

gebildet sind.

2. Verfahren nach Anspruch 1, dadurch gekennzeichnet, daß die Katalysatoren aus Heteropolysäuren bzw. deren dehydratisierten Derivaten der Formel

$$Cu_aH_bP_cMo_dV_eO_f \qquad\qquad (I)$$

mit

a : 0,1 bis 1, b = 0 bis 7,8, c = 0,8 bis 1,2, vor allem 1, d = 9 bis 12, vor allem 10 oder 11, e = 0,5 bis 3, vor allem 1 oder 2 und f = abhängig von den Molzahlen a bis e gebildet sind.

3. Verfahren nach Anspruch 1, dadurch gekennzeichnet, daß der Katalysator aus der Heteropolysäure der Formel

$$H_8PMo_{10}VO_{39} \qquad\qquad (II)$$

bzw. deren Anhydrid

$$PMo_{10}VO_{35} \qquad\qquad (III)$$

gebildet ist.

4. Verfahren nach den Ansprüchen 1 bis 3, dadurch gekennzeichnet, daß die Oxidation von Isobutan in Gegenwart von Inertgasen, wie insbesondere Stickstoff, Kohlendioxid und/oder Wasserdampf durchgeführt wird.

5. Verfahren nach den Ansprüchen 1 bis 4, dadurch gekennzeichnet, daß Methacrolein zusammen mit Isobutan in den Reaktor zum Katalysator geführt wird.


**Claims**

1. A process for preparing methacrylic acid and methacrolein through oxidation of isobutane with molecular oxygen in the gaseous phase, at temperatures of from 250 to 450°C, on a fixed catalyst prepared from heteropolyacids which contain phosphorus as a central element and molybdenum as a co-ordinating element, characterised in that the catalysts are formed from heteropolyacids or the dehydrated derivatives thereof of

formula

$$Cu_aH_bP_cMo_dV_eO_f \qquad (I)$$

wherein

a = 0.1 to 1, b = 0 to 7.8, c = 0.8 to 1.2, d = 9 to 12, e = 0.5 to 3, f is dependent on the molar numbers a to e, or/and of formula

$$H_8PMo_{10}VO_{39} \qquad (II)$$

or the anhydride thereof

$$PMo_{10}VO_{35} \qquad (III)$$

2. A process according to claim 1, characterised in that the catalysts are formed from heteropolyacids or the dehydrated derivatives thereof of formula

$$Cu_aH_bP_cMo_dV_eO_f \qquad (I)$$

wherein

a : 0.1, to 1, b = 0 to 7.8, c = 0.8 to 1.2, especially 1, d = 9 to 12, especially 10 or 11, e = 0.5 to 3, especially 1 or 2 and f is dependent on the molar numbers a to e.

3. A process according to claim 1, characterised in that the catalyst is formed from the heteropolyacid of formula

$$H_8PMo_{10}VO_{39} \qquad (II)$$

or the anhydride thereof

$$PMo_{10}VO_{35} \qquad (III)$$

4. A process according to claims 1 to 3, characterised in that the oxidation of isobutane is carried out in the presence of inert gases such as especially nitrogen, carbon dioxide and/or water vapour.

5. A process according to claims 1 to 4, characterised in that methacrolein is introduced onto the catalyst in the reactor, together with isobutane.


**Revendications**

1. Procédé de préparation d'acide méthacrylique et de méthacroléine par oxydation d'isobutane avec de l'oxygène moléculaire, en phase gazeuse, à des températures de 250 à 450°C et en présence d'un catalyseur solide préparé à partir d'hétéropolyacides qui contiennent du phosphore en tant qu'élément central et du molybdène en tant qu'élément de coordination, caractérisé en ce que les catalyseurs sont formés à partir d'hétéropolyacides ou de leurs dérivés déshydratés de formule

$$Cu_aH_bP_cMo_dV_eO_f \qquad (I)$$

avec

a = 0,1 à 1, b = 0 à 7,8, c = 0,8 à 1,2, d = 9 à 12, e = 0,5 à 3, f = dépend du nombre de moles a à e, et/ou de formule

$$H_8PMo_{10}VO_{39} \qquad\qquad\qquad (II)$$

ou de son anhydride

$$PMo_{10}VO_{35} \qquad\qquad\qquad (III)$$

2. Procédé selon la revendication 1, caractérisé en ce que les catalyseurs sont formés à partir d'hétéropolyacides ou de leurs dérivés déshydratés de formule

$$Cu_aH_bP_cMo_dV_eO_f \qquad\qquad\qquad (I)$$

avec
a = 0,1 à 1, b = 0 a 7,8, c = 0,8 à 1,2, avant tout 1, d = 9 à 12, avant tout 10 ou 11, e = 0,5 à 3, avant tout 1 ou 2 et f = dépend du nombre de moles a à e,

3. Procédé selon la revendication 1, caractérisé en ce que le catalyseur est formé à partir de l'hétéropolyacide de formule

$$H_8PMo_{10}VO_{39} \qquad\qquad\qquad (II)$$

ou de son anhydride

$$PMo_{10}VO_{35} \qquad\qquad\qquad (III)$$

4. Procédé selon l'une quelconque des revendications 1 à 3, caractérisé en ce que l'oxydation d'isobutane est conduite en présence de gaz inertes, tels en particulier que l'azote, l'anhydride carbonique et/ou la vapeur d'eau.

5. Procédé selon l'une quelconque des revendications 1 à 4, caractérisé en ce que de la méthacroléine est envoyée sur le catalyseur dans le réacteur, en même temps que l'isobutane.